Europäisches Patentamt

European Patent Office

Office européen des brevets

(11) Veröffentlichungsnummer : **0 105 870**
**B1**

(12) # EUROPÄISCHE PATENTSCHRIFT

(45) Veröffentlichungstag der Patentschrift :
11.02.87

(21) Anmeldenummer : 83890173.4

(22) Anmeldetag : 03.10.83

(51) Int. Cl.⁴ : **G 01 N 21/64,** A 61 B  5/14,
**G 01 N 31/22**

(54) **Messeinrichtung zur Bestimmung des CO2-Gehaltes einer Probe.**

(30) Priorität : 06.10.82 AT 3700/82
16.09.83 AT 3314/83

(43) Veröffentlichungstag der Anmeldung :
18.04.84 Patentblatt 84/16

(45) Bekanntmachung des Hinweises auf die Patenterteilung : 11.02.87 Patentblatt 87/07

(84) Benannte Vertragsstaaten :
DE FR GB

(56) Entgegenhaltungen :
EP-A- 0 016 387
EP-A- 0 083 703
DE-A- 2 117 290
DE-A- 2 508 637
DE-B- 2 823 318
US-A- 3 612 866

(73) Patentinhaber : **AVL AG**
**Grabenstrasse 11**
**CH-8201 Schaffhausen (CH)**

(72) Erfinder : **Marsoner, Hermann**
**Jakominiplatz 17**
**A-8010 Graz (AT)**
Erfinder : **Kroneis, Herbert**
**Jakob Gschiel-Gasse 8/21**
**A-8052 Graz (AT)**
Erfinder : **Wolfbeis, Otto**
**Schöckelbachweg 37b**
**A-8045 Graz (AT)**

(74) Vertreter : **Krause, Walter, Dr. Dipl.-Ing.**
**Postfach 200 Singerstrasse 8**
**A-1014 Wien (AT)**

EP 0 105 870 B1

## Beschreibung

Die Erfindung betrifft eine Meßeinrichtung zur Bestimmung des $CO_2$-Gehaltes einer Probe, mit einem pH-abhängigen Fluoreszenzfarbstoff als Indikator, der in wäßriger Lösung in einer protonendichten, gasdurchlässigen Polymermembran eingebettet und mit dem die Probe zumindest teilweise in Kontakt bringbar ist, und einer Anordnung zur Messung des vom Indikator nach Anregung abgegebenen Fluoreszenzlichtes.

Es ist bekannt, daß die Messung des Partialdruckes von $CO_2$ ($PCO_2$) in Flüssigkeiten oder Gasen auf eine pH-Messung zurückgeführt werden kann. Dazu ist im allgemeinen ein Reaktionsraum erforderlich, in welchem der pH-Wert detektiert wird, der durch den jeweiligen $PCO_2$-Wert des Meßgutes bestimmt wird :

$$pH = pKs + \log \frac{HCO_3}{\alpha . PCO_2}$$

Dabei muß gewährleistet sein, daß der pH-Wert des Reaktionsraumes durch den aktuellen pH-Wert des zu messenden Mediums nicht beeinflußt wird, wohl aber ein Austausch von $CO_2$ zwischen Meßgut und Reaktionsraum ermöglicht wird, welcher zu einem Gleichgewichtszustand für $CO_2$ zwischen Meßgut und Reaktionsraum führt.

Üblicherweise wird diese Forderung dadurch erfüllt, daß Meßgut und Reaktionsraum durch eine gasdurchlässige, protonenundurchlässige Membran (Teflon, Silicon) voneinander getrennt werden.

$PCO_2$-Meßsysteme dieser Art, die sich zur pH-Messung im Reaktionsraum einer Glaselektrode bedienen, wurden als « Severinghous-Elektroden » bekannt und werden vielfach eingesetzt, so zum Beispiel auch in der Blutgasanalyse.

Es ist weiters bekannt, daß die Detektion des pH-Wertes in $PCO_2$-Meßsystemen dieser Art auch mit Hilfe von pH-abhängigen Fluoreszenzfarbstoffen auf optischem Wege durchgeführt werden kann. (« $PCO_2$-Optoden »). Als Indikatoren können prinzipiell alle fluoreszierenden Moleküle mit dissoziationsfähigen Gruppen angesehen werden. Solche Indikatoren sind z. B. : Chinin, Harman, Harmin, Harmol, Pyronin G, Thiochrom, Luminol, 1-Naphthol-2-sulfonat, 1-Naphthol-4-sulfonat, 2-Naphthol-6-sulfonat, Umbelliferon, 4-Methyl-umbelliferon, Fluorescein, 1,3-Dihydroxypyren-6, 6-trisulfonat, 7-Hydroxyflavon, 7-Hydroxyisoflavon, Ortho-Cumarsäure, Salicylaldehydsemicarbazon, 3-Hydroxyxanthon, 3,6-Dihydroxyxanthon, 7-Hydroxylapidon, 3-Hydroxyacridon, Harmolmethojodid, 7-Hydroxy-2-methyl-chromon.

In solchen $PCO_2$-Optoden ist der Reaktionsraum gleichzeitig Indikatorraum. Der flache Reaktions- bzw. Indikatorraum wird auf der einen Seite durch eine gasdurchlässige, protonendichte Membran begrenzt, die ihrerseits an das Meßgut angrenzt, auf der anderen Seite durch eine lichtdurchlässige Fläche, durch welche in bekannter Weise (Lichtquelle, Monochromatoren, Lichtempfänger) die Fluoreszenzlichtintensität gemessen wird. Die Intensität des Fluoreszenzlichtes ist dabei eine Funktion des pH-Wertes im Reaktionsraum, welcher durch den $PCO_2$ im Meßgut bestimmt wird.

Weiters ist auch bekannt, daß der Indikatorraum vorteilhafterweise aus einer Folie bestehen kann, in welcher der Indikator leckfrei eingesiegelt ist. Durch eine solche Einsiegelung wirkt die Folie selbst als selektive Membran, die gleichzeitig gegen die Flüssigkeit absperrt, sowie als optisch durchstrahlbarer Indikatorraum. Der Vorteil dieser Anordnung besteht in dem einfachen und robusten Aufbau der gesamten Meßeinrichtung.

Als entscheidender Nachteil dieser Anordnung ist folgendes anzusehen :

Wie immer geartetes Folienmaterial ist polymer aufgebaut. Im folgenden sei zwischen hydrophilen und hydrophoben Polymeren unterschieden :

Besteht die Folie aus einem hydrophilen Träger, kann keine $CO_2$-Selektivität vorliegen ; — das Folienmaterial ist protonendurchlässig. Darüberhinaus ist ein Ausschwemmen von wasserlöslichen Indikatormolekülen zu erwarten. Besteht die Folie aus einem hydrophoben Polymer, kann innerhalb der Folie aufgrund der Abwesenheit von Wasser kein pH-Wert gemessen werden.

Die Herstellung einer einfachen und robusten $PCO_2$-Meßeinrichtung blieb also ein offenes Problem, so daß zunächst ein System vorhanden war, bei dem der Indikatorraum aus einer wäßrigen Indikatorlösung bestand, welche vom Meßgut durch eine hydrophobe Membran getrennt war. Die Nachteile dieses Systems liegen einerseits in der Schwierigkeit der reproduzierbaren Herstellung, andererseits in seiner mechanischen Instabilität. Eine Variation der Schichtstärke oder ungleichmäßige Schichtstärkenverteilung nehmen Einfluß auf die optischen Eigenschaften und damit auf die Empfindlichkeit des Meßsystems. Zeitliche Veränderungen dieser Eigenschaften sind zu erwarten, besonders dann, wenn das System mechanischen oder thermischen Belastungen ausgesetzt wird, wie sie etwa die Verwendung in einem automatischen Analysengerät (Reinigungsvorgänge, Anlegen von Saugdruck) mit sich bringt.

Eine weitere Möglichkeit zur Stabilisierung von $PCO_2$-Optoden besteht prinzipiell darin, kleinste Kapseln, gefüllt mit wäßriger Indikatorlösung, herzustellen und diese Kapseln möglichst homogen verteilt in ein hydrophobes, optisch durchstrahlbares Membranmaterial einzubetten.

Ein Verfahren zur Herstellung von Mikrokapseln im Nanometergrößenbereich wurde z. B. in der DE-OS 23 60 384 beschrieben. Eine wäßrige Lösung von Wirkstoffen (z. B. von Indikatoren) und polymerisationsfähigen Molekülen wird mit Hilfe von Tensiden in einer hydrophoben Flüssigkeit durch Rühren verteilt. Dabei entstehen kleinste Tröpfchen von Wirkstoff und polymerisationsfähigen Molekülen in der hydrophoben Phase, deren Polymerisation zu Kapseln im Nanometergrößenbereich führt. Dem Umstand, daß es dabei bisher nicht gelang, ein selektives (also protonenimpermeables) Kapselwandmaterial für PCO₂-Kapseln (darunter sind hier und im folgenden Kapseln, gefüllt mit entsprechender Indikatorsubstanz zu verstehen) herzustellen, wird dadurch Rechnung getragen, daß die Kapseln in ein selektiv wirkendes Folienmaterial eingebettet werden.

Weitere in diesem Zusammenhang auftretende Nachteile sind : Die Herstellung von derartigen PCO₂-Kapseln ist anfällig auf kleine Änderungen in der Versuchsführung. Während des Herstellungsprozesses und bei der Weiterverarbeitung dieser Kapseln werden häufig irreversible Kapselagglomerationen beobachtet, wodurch eine gleichmäßige Verteilung in einem Träger unmöglich wird.

Eine Optimierung dieses zuletzt genannten bekannten Verfahrens (Herstellung und Einbettung von PCO₂-Kapseln in Polymerfolien) könnte zwar prinzipiell zu robusten PCO₂-Optoden führen, die Nachteile von hohem Kosten- und Zeitaufwand sowie Schwierigkeiten bei der Reproduzierbarkeit des Herstellungsverfahrens blieben aber bestehen.

Weiters ist es aus der DE-OS 25 08 637 bekannt, Trägerpartikel zu verwenden, die einen eingesiegelten Indikator enthalten und die zusammen mit dem zu messenden Stoff und einer Trägerflüssigkeit, beispielsweise Blut, eine von monochromatischem Licht durchstrahlte Durchflußkammer durchfließen. Nachteilig dabei sind u. a. der durch die Beimengung der Trägerteilchen entstehende Handhabungsaufwand bzw. die Meßungenauigkeiten, sowie daß hier eine mechanisch nicht stabile Optode vorliegt.

Aufgabe der Erfindung ist es, eine PCO₂-Meßeinrichtung der eingangs genannten Art so zu verbessern, daß die angeführten Nachteile der bekannten Einrichtungen vermieden sind und daß insbesonders die Optode (also Träger samt Indikator) einfach, kostengünstig und reproduzierbar herstellbar ist. Weiters soll die PCO₂-Optode mechanisch stabil aufgebaut sein.

Dies wird gemäß der Erfindung dadurch erreicht, daß die wäßrige Indikatorlösung in Form von Tröpfchen mit einem Durchmesser in der Größenordnung von 0,1-100 μm in gleichmäßiger Verteilung in der Polymermembran eingebaut vorliegt.

Der Aufbau einer mechanisch stabilisierten PCO₂-Meßeinrichtung gelang also dadurch, daß Mizellen (so seien hier und im folgenden in Anlehnung an eine in anderem Zusammenhang gebräuchliche Bezeichnung die genannten Tröpfchen bezeichnet) einer wäßrigen Indikatorlösung in gleichmäßiger Verteilung in eine geeignetes protonendichtes Membranmaterial eingebaut sind.

Für die Herstellung dieser Art von PCO₂-Optoden mußten an das verwendete Polymer und an die Ausgangsgemische desselben bestimmte Anforderungen gestellt werden.

a) Anforderungen an das Polymer :
— Es muß im spektralen Bereich von Anregung und Emission des Indikators lichtdurchlässig sein.
— Es muß selektive Eigenschaften haben ; — im speziellen muß es permeabel für Gase wie CO₂ sein und impermeabel für Ionen wie Protonen, Oxonium- und Hydroniumionen oder Salze, sowie impermeabel für makromoleküle Bestandteile eine Meßgutes.

b) Anforderungen an die Ausgangsgemische des Polymers (Unter Ausgangsgemisch sind entweder Reaktionsgemische zu verstehen, aus welchen durch Polymerisation, Polykondensation, Polyaddition oder Vernetzung bzw. Vulkanisation Polymere hergestellt werden oder auch eine Lösung von Polymeren in entsprechenden Lösungsmitteln, wobei das polymere Endprodukt durch Verdunstung dieser Lösungsmittel entsteht. Alle Methoden zum Überführen von Ausgangsgemischen in polymere Endprodukte werden im folgenden Polymerisation genannt).
— Es muß möglich sein, eine für die Dauer der Herstellung stabile Emulsion von Wasser im Polymerausgangsprodukt zu erzeugen (die Emulsion muß bis zur Beendigung der Polymerisation stabil sein).
— Die Anwesenheit von Wasser darf weder zu Polymerisationen führen, noch eine spätere Polymerisation beeinflussen.
— Die für die Herstellung der Emulsion erforderlichen Bedingungen (z. B. Rühren und Wärmeentwicklung dabei) dürfen weder zur Polymerisation führen, noch eine solche beeinflussen.
— Es muß möglich sein, die Emulsion vor der Polymerisation in eine geeignete Form (Folie, Membran) überzuführen. Es ist im Prinzip auch möglich, eine Emulsion Blocks auszupolymerisieren und davon durch geeignete Methoden flächenförmige Optoden anzuschneiden. Dieses Verfahren bringt allerdings Nachteile mit sich :
1. Da einige Mizellen zerschnitten werden, entstehen für optische Messungen nachteilige inhomogene Oberflächen.
2. Eine Fixierung der Polymerfolie auf einem transparenten Träger, die vorteilhafterweise zur mechanischen Stabilisierung durchgeführt werden kann, stellt einen zusätzlichen Arbeitsschritt dar.
— Allfällig entstehende, flüchtige Polymerisationsprodukte dürfen keinen Einfluß auf die Eigenschaften der indikatorhältigen Mizellen haben, so darf z. B. der pH-Wert der Mizellen nicht verändert werden.

Wegen der vorgenannten Anforderungen können beispielsweise hydrophile oder nichttransparente Polymere nicht verwendet werden. Ebenso scheiden Polymerlösungen, sowie Polymere aus, welche bei hoher Temperatur oder hohem Druck polymerisiert werden.

Überraschenderweise konnte aus der Gruppe der Silicone ein Vertreter gefunden werden, welcher den Anforderungen entspricht und die Herstellung von Membranen erlaubte, welche Indikatorlösung in emulgierter Form und homogener Verteilung enthielt.

Gemäß einer vorteilhaften Ausgestaltung der Erfindung ist demgemäß vorgesehen, daß die Polymermembran aus Siliconkautschuk, besteht.

Obwohl Silicone die geforderten Eigenschaften, nämlich Protonenimpermeabilität, Gaspermeabilität und Transparenz aufweisen, war zunächst ein Einsatz dieser Polymerklasse nicht naheliegend. Üblicherweise wird die Vulkanisation von Silicon nämlich durch Wasser entweder initiiert oder inhibiert.

Bei den für die Herstellung einer derartigen Silicontype beispielsweise verwendeten Reagenzien handelt es sich um Produkte der Wacker-Chemie G.m.b.H., Burghausen, BRD, mit den Handelsbezeichnungen SLM 40060, SLM 40061 und SLM 40062 (Vorpolymer, Katalysator, Härter). Das nachstehende Endprodukt ist ein additionsvernetzter Zweikomponentenkautschuk, dessen Vulkanisierung einerseits durch anwesendes Wasser nicht gestört wird und andererseits nur durch den Einflüß von Lichtenergie bestimmter Wellenlänge abläuft. Unter optimalen Bedingungen ist die Vulkanisation innerhalb weniger Sekunden abgeschlossen.

Bestimmt durch die Eigenschaften des gewählten Polymermaterials kann die Herstellung von robusten $PCO_2$-Optoden bzw. Meßeinrichtungen in einfacher und reproduzierbarer Weise durchgeführt werden :

Zunächst werden die erforderlichen Komponenten für das Polymermaterial vermischt. In der Folge wird wäßrige Indikatorlösung zugesetzt und durch Rühren eine Emulsion von Indikatorlösung im Ausgangsgemisch für das Polymer hergestellt. Die Emulsion wird durch Ausstreichen, Ausgießen oder andere geeignete Verfahren in dünner Schicht auf eine glatte Oberfläche aufgebracht und in dieser ausgesetzt.

Um eine Meßeinrichtung nach der vorliegenden Erfindung dahingehend weiterzubilden, daß gleichzeitig bzw. zumindest mit der gleichen Anordnung zusätzliche Meßwerte an der Probe bestimmbar sind, ist in einer vorteilhaften Ausgestaltung vorgesehen, daß die Polymermembran von einem polymerisierten Siliconpräpolymer gebildet ist, in dem zusätzlich zu den pH-abhängig fluoreszierenden Indikatortröpfchen zumindest annähernd homogen verteilte, $O_2$-abhängig fluoreszierende Indikatorsubstanz in solubilisierter Form gebunden vorliegt, und daß die beiden Indikatoren unterschiedliche Emissionswellenlängen aufweisen. Auf diese sehr einfache Weise ist eine Einrichtung geschaffen, die neben der Bestimmung des $CO_2$- auch die des $O_2$-Gehaltes ermöglicht. Die Reaktion auf die Anwesenheit von $CO_2$ ist dabei völlig unabhängig von der Reaktion auf die Anwesenheit von $O_2$, da die beiden Indikatoren räumlich und auch chemisch voneinander getrennt vorliegen. Voraussetzung für die tatsächliche Unabhängigkeit der

Sensorfunktionen für $O_2$ bzw. $CO_2$ ist dabei noch, daß die Emissionswellenlänge der beiden verwendeten Indikatoren einen ausreichend großen Unterschied aufweisen, sodaß das Fluoreszenzlicht durch übliche optische Filter voneinander separiert werden kann.

Aus einem beispielsweise eindimensionalen Silicon-Präpolymer mit niedrigem Molekulargewicht, welches leicht verarbeitbar ist, entsteht durch eine weitere Polymerisation ein mehrfach vernetztes widerstandsfähiges Siliconpolymer.

Vorteilhaft ist dabei die Verwendung von Dehäsivsilicon. Sogenannte Dehäsivsilicone gehen im Gegensatz zu Adhäsivsiliconen keine Verbindung mit Glas unter Freisetzung von Essigsäure ein, welche irreversible, nachteilige Veränderungen in der Polymermembran verursachen kann. Dehäsivsilicone haften zwar nicht so gut auf Glas wie Adhäsivsilicone ; die Haftung ist jedoch für die gewünschte Anwendung ausreichend.

Im Hinblick auf den zusätzlichen Einbau der $O_2$-abhängig fluoreszierenden Indikatorsubstanz in solubilisierter Form ist zu erwähnen, daß der Einbau von in Frage kommenden Indikatorstoffen in Silicon-Polymere dahingehend Schwierigkeiten bereitet, daß die Konzentration der Indikatorstoffe, die durch die üblichen Verfahren eingebracht werden können, für ausreichende Fluoreszenzsignalhöhe, sodaß diese meßtechnisch brauchbar wird, unzureichend gering ist. Es hat sich jedoch überraschenderweise herausgestellt, daß sich die in Frage kommenden Indikatorsubstanzen chemisch modifizieren, nämlich solubilisieren lassen, sodaß ausreichend hohe Konzentrationen der Indikatorsubstanzen in Silicon in Lösung gebracht werden können.

Unter Solubilisierung (« löslich machen ») ist hier zu verstehen, daß die Löslichkeit einer Substanz in einem (auch polymeren) Lösungsmittel durch Modifizierung der Substanz (chemische Modifizierung) erhöht wird.

Die Veränderung an den Indikatorsubstanzen erfolgt im wesentlichen analog der an sich bekannten Friedel Crafts Alkylierung an Aromaten.

Es hat sich damit gezeigt, daß trotz Erhöhung der Löslichkeit des fluoreszierenden Stoffs weitestgehend ungestörtes Löschverhalten vorliegt, wenn die folgenden, an sich bekannten Schritte durchgeführt werden.

1. Indikator und tert. Butylchlorid werden in einem geeigneten Lösungsmittel ($CS_2$) gelöst und unter dem katalytischen Einfluß von Aluminiumchlorid umgesetzt.

2. Nach einem Extraktionsvorgang folgen Wasch- und Trockenprozesse ; — ein Entfernen von überschüssigen organischen Lösungsmitteln durch Rotationsverdampfen führt zu einem ölartigen Rückstand, welcher direkt als « solubilisierter Indikator » eingesetzt werden kann, oder : wie oben, aber der Indikator wird in einem Überschuß von tert. Butylchlorid ohne Zusatz eines weiteren Lösungsmittels gelöst.

Durch diese geschilderten Maßnahmen können nun zu Membranen verarbeitbare Polymer- bzw. Präpolymergemische mit sauerstoffempfindlichen Indikatorsubstanzen hergestellt werden, wobei die Konzentration des Indikators in diesen Gemischen so hoch ist, daß auch in dünner Schicht (z. B. unterhalb von 50 μ Schichtstärke) meßtechnisch ausreichende Signalhöhen erreicht werden können. Die Weiterverarbeitung der Polymergemische erfolgt durch übliche Verfahren zu dünnen Membranen. Diese Verfahren können Ausstreichen, Gießen oder andere Verfahren beinhalten, wie sie zum Beschichten von Oberflächen mit Polymeren angewandt werden. Ein weiterer Vorteil dieser Vorgangsweise ist, daß während des Polymerisationsvorgangs eine dünne Membran auf einem festen Trägermaterial in haftender Verbindung aufgebracht werden kann.

Auf Träger fixierte dünne Polymermembranen mit $O_2$-Indikatorsubstanzen der beschriebenen Art konnten zur zusätzlichen fluoreszenzphotometrischen Messung von Sauerstoff in Gas angewandt werden. Dabei hat sich als besonderer Vorteil dieser Meßtechnik gezeigt, daß man Einstellzeiten von reinem Stickstoff auf reinen Sauerstoff in der Größenordnung bis zu 0,15 sec. erreichen konnte.

Obwohl allein das Auflösen von Indikatoren im polymeren Trägermaterial häufig ausreicht, um Indikatorverluste an die Umgebung zu verhindern, kann es für verschiedene Anwendungszwecke von Vorteil sein, andere Methoden zur Immobilisierung von Substanzen in Polymeren anzuwenden.

Diese sind z. B.

a) Einschränkung der Indikatorbeweglichkeit im Polymer durch chemische Modifizierung (Alkylierung mit längeren C-Ketten) der Indikatoren

b) Kovalente Bindung der Indikatoren an die Polymersubstanz.

Als $O_2$-abhängig fluoreszierende Indikatorsubstanz können gemäß einer vorteilhaften Ausgestaltung der Erfindung polyzyklische, homo- oder heterozyklische aromatische Moleküle, vorzugsweise polyzyklische aromatische Kohlenwasserstoffe mit Fluoreszenzabklingzeiten größer als 5 ns verwendet werden. Dabei ist es weiters vorteilhaft, wenn die $O_2$-abhängig fluoreszierende Indikatorsubstanz zur Immobilisierung in chemisch modifizierter Form vorliegt, vorzugsweise mit längeren verzweigten oder unverzweigten Kohlenwasserstoffketten von $C_3$ bis $C_{20}$ alkyliert, wodurch die Indikatorbeweglichkeit im Siliconpolymer eingeschränkt ist.

Vorteilhafterweise ist in einer weiteren Ausgestaltung der Erfindung vorgesehen, daß die Polymermembran auf eine glatte Oberfläche eines transparenten, schichtförmigen Trägermaterials aufvulkanisiert bzw. aufpolymerisiert ist. Das transparente Trägermaterial kann beispielsweise Glas oder Vinylglas sein. Der Vorteil besteht darin, daß die Membran auf der Fläche haften bleibt. Die Haftfähigkeit kann darüberhinaus durch geeignete Haftvermittler zusätzlich verbessert werden.

Das transparente Trägermaterial erfüllt zweierlei Funktionen : Es begrenzt die Optode gegen die Fluoreszenzlichtmeßeinrichtung hin und trägt zur mechanischen Stabilisierung der Membran bei.

In Weiterbildung der Erfindung sind im Polymermembranmaterial Platzhalter für die indikatorhaltigen Mizellen vorgesehen, die die Indikatorlösung entweder an der Oberfläche tragen oder von dieser durchdrungen sind. Dadurch wird die Verformbarkeit der Mizellen unter Einwirkung äußerer Kräfte reduziert.

Solche Platzhalter, welche die gleiche Größenordnung wie die Tröpfchen bzw. Mizellen haben, können aufgrund ihres Aufbaues oder ihrer Zusammensetzung wasseranziehende Eigenschaften haben (z. B. Kieselgel oder Polyacrylamid), wodurch ein Austrocknen der Membrane bzw. Optode verhindert oder zumindest verzögert wird. Darüberhinaus wird ein Wiederbefeuchten der Mizellen erleichtert und beschleunigt.

Das Einbringen solcher Platzhalter, welche nach einem weiteren Merkmal der Erfindung auch von Kügelchen aus porösem Material, z. B. Glaskügelchen, gebildet sein können, in das Polymermembranmaterial kann auf zweierlei Weise erfolgen :

1. Die die Indikatorlösung tragenden Platzhalter werden, vorzugsweise einschichtig, auf einen transparenten Träger aufgebracht, welcher die Optode zur Lichtmeßeinrichtung hin abschließt. Auf diese Schicht wird das Ausgangsgemisch für das Polymer aufgebracht und vulkanisiert.

2. Die die Indikatorlösung tragenden Platzhalter werden durch Rühren in das Ausgangsgemisch für das Polymer eingebracht und gleichmäßig verteilt. Das Verfahren der Weiterverarbeitung entspricht jenem für Emulsionen ohne Platzhalter.

Zur Verhinderung der mechanischen Zerstörung von oberflächennahen Mizellen mit oder ohne Platzhalter kann in Weiterbildung der Erfindung an der der Probe zugewandten Seite der Polymermembran eine vorzugsweise dicht aufpolymerisierte Schutzmembran aufgebracht sein.

Diese zusätzliche Schutzmembran kann vorteilhafterweise lichtundurchlässig sein, bzw. durch geeignete Methoden lichtundurchlässig gemacht werden (z. B. durch Einlagerung von Pigmenten oder Farbstoffen). Dadurch wird die eigentliche Optodenmembran vor oberflächlicher Zerstörung geschützt und gleichzeitig von der Probe optisch isoliert.

Die in den Mizellen vorhandene Indikatorlösung (mit oder ohne Platzhalter) kann nach einer anderen Weiterbildung der Erfindung ein zusätzliches Puffersyste, z. B. Bikarbonat- oder Phosphatpuffer enthalten. Solche Zusätze sind aber nicht zwingend erforderlich, da Indikatoren selbst als Puffersystem wirken, und durch geeignete Wahl der Indikatorkonzentration eine auf die Meßeinrichtung abgestimmte, geeignete Pufferkapazität eingestellt werden kann.

Beispiele für die Herstellung einer PCO$_2$-Optode für eine Meßeinrichtung nach der Erfindung :

1. 10 g SLM 40060 werden mit 40 mg SLM 40061 und 400 mg SLM 40062 vermischt. Zu diesem Gemisch wird 1 g einer 10$^{-2}$ molaren wäßrigen Lösung von Pyranin zugesetzt, und eine Emulsion durch Rühren mit einem Schnellrührer hergestellt. Die Rührzeit beträgt ca. 30 sec. Diese Emulsion wird mit Hilfe einer Ausstreichvorrichtung in einer Schichtdecke von ca. 50 μm auf einen entfetteten Glasträger aufgebracht. Die Schicht wird durch Einwirkung von ultravioletter Strahlung vulkanisiert.

2. Zu 15 ml einer 10$^{-2}$ molaren, wäßrigen Lösung von Pyranin werden 2 g von Kügelchen aus quervernetztem Polyacrylamid mit einem mittleren Durchmesser von 20 μm zugesetzt. Die mit Indikatorlösung getränkten, als Platzhalter wirkenden Kügelchen werden abgenutscht und in einem Gewichtsverhältnis von 5 : 1 zugunsten des Polymerausgangsgemisches mit letzterem vermischt. Die Weiterverarbeitung entspricht jener in Beispiel 1.

Beispiel für die Herstellung einer erfindungsgemäßen Meßeinrichtung zur Bestimmung von CO$_2$ und O$_2$ :

1. 0,1 g solubilisiertes Bezo(ghi) perylen werden in 10 g SLM 40060 (Wacker Chemie, BRD) gelöst.

2. Zu 15 ml einer 0,01 molaren, wäßrigen Lösung von Pyranin werden 2 g von Kügelchen aus quervernetztem Polyacrylamid mit einem mittleren Durchmesser von 20 μm zugesetzt. Die mit Indikatorlösung getränkten, als Platzhalter wirkenden Kügelchen werden abgenutscht und in einem Gewichtsverhältnis von 1 : 5 in folgendes Polymerausgangsgemisch eingerührt (1 Teil Kügelchen, 5 Teile Polymerausgangsgemisch) :

10 g SLM 40060 mit solubilisiertem Benzo(ghi)perylen
40 mg SLM 40061
400 mg SLM 40062

3. Diese Suspension wird mit Hilfe einer Streichvorrichtung in einer Schichtdicke von etwa 50 μm auf einen entfetteten Glasträger aufgebracht. Die Schicht wird durch Einwirkung von ultravioletter Strahlung vulkanisiert.

Das in der hydrophoben Membranmatrix gelöste solubilisierte Benzo(ghi)perylen wirkt als P$_2$-Indikator. Das im wäßrigen Inhalt der suspendierten Kügelchen gelöste Pyranin wirkt als CO$_2$-Indikator.

## Patentansprüche

1. Meßeinrichtung zur Bestimmung des CO$_2$-Gehaltes einer Probe, mit einem pH-abhängigen Fluoreszenzfarbstoff als Indikator, der in wäßriger Lösung in einer protonendichten, gasdurchlässigen Polymermembran eingebettet und mit dem die Probe zumindest teilweise in Kontakt bringbar ist, und einer Anordnung zur Messung des vom Indikator nach Anregung abgegebenen Fluoreszenzlichtes, dadurch gekennzeichnet, daß die wäßrige Indikatorlösung in Form von Tröpfchen mit einem Durchmesser in der Größenordnung von 0,1-100 μm in gleichmässiger Verteilung in der Polymermembran eingebaut vorliegt.

2. Meßeinrichtung nach Anspruch 1, dadurch gekennzeichnet, daß die Polymermembran aus Siliconkautschuk besteht.

3. Meßeinrichtung nach Anspruch 1, dadurch gekennzeichnet, daß die Polymermembran von einem polymerisierten Siliconpräpolymer gebildet ist, in dem zusätzlich zu den pH-abhängig fluoreszierenden Indikatortröpfchen zumindest annähernd homogen verteilte, O$_2$-abhängig fluoreszierende Indikatorsubstanz in solubilisierter Form gebunden vorliegt, und daß die beiden Indikatoren unterschiedliche Emissionswellenlängen aufweisen.

4. Meßeinrichtung nach Anspruch 3, dadurch gekennzeichnet, daß polyzyklische homo- oder heterozyklische aromatische Moleküle, vorzugsweise polyzyklische aromatische Kohlenwasserstoffe mit einer Fluoreszenzabklingzeit $\tau_O$ größer als 5 ns, als O$_2$-abhängig fluoreszierende Indikatorsubstanz verwendet sind.

5. Meßeinrichtung nach Anspruch 3 oder 4, dadurch gekennzeichnet, daß die O$_2$-abhängig fluoreszierende Indikatorsubstanz zur Immobilisierung in chemisch modifizierter Form vorliegt, vorzugsweise mit längeren verzweigten oder unverzweigten Kohlenwasserstoffketten von $C_3$ bis $C_{20}$ alkyliert, wodurch die Indikatorbeweglichkeit im Siliconpolymer eingeschränkt ist.

6. Meßeinrichtung nach einem der Ansprüche 1 bis 5, dadurch gekennzeichnet, daß die Polymermembran auf eine glatte Oberfläche eines transparenten, schichtförmigen Trägermaterials aufvulkanisiert bzw. aufpolymerisiert ist.

7. Meßeinrichtung nach einem der Ansprüche 1 bis 6, dadurch gekennzeichnet, daß in Polymermembranmaterial Platzhalter für die indikatorhaltigen Tröpfchen, welche in der gleichen Größenordnung wie diese sind, vorgesehen sind, die die Indikatorlösung entweder an der Oberfläche tragen oder von dieser durchdrungen sind.

8. Meßeinrichtung nach Anspruch 7, dadurch gekennzeichnet, daß die Platzhalter von Kügelchen bzw. kleinsten Teilen aus hygroskopischem Material, wie beispielsweise Kieselgel oder Polyacrylamid, gebildet sind.

9. Meßeinrichtung nach Anspruch 7, dadurch gekennzeichnet, daß die Platzhalter von Kügelchen aus porösem Material z. B. Glaskügelchen, gebildet sind.

10. Meßeinrichtung nach einem der Ansprüche 1 bis 9, dadurch gekennzeichnet, daß an der der Probe zugewandten Seite der Polymermembran eine vorzugsweise dicht aufpolymerisierte Schutzmembran aufgebracht ist.

11. Meßeinrichtung nach Anspruch 10, dadurch gekennzeichnet, daß die der Anordnung zur Messung des Fluoreszenzlichtes abgewandte Schutzmembran lichtundurchlässig ist.

12. Meßeinrichtung nach einem der Ansprüche 1 bis 11, dadurch gekennzeichnet, daß die Indikatorlösung ein zusätzliches Puffersystem, vorzugsweise Bikarbonat- oder Phosphatpuffer, enthält.

## Claims

1. A measuring device for determining the content of $CO_2$ in a sample, comprising as an indicator a pH-dependent fluorescent dye in aqueous solution which is embedded in a proton-impervious, gas-permeable polymer membrane and with which the sample may be brought into contact at least partially, and further comprising an arrangement for measuring the fluorescent light emitted by the indicator upon excitation, wherein the aqueous indicator solution is incorporated in the polymer membrane in homogeneous dispersion in the form of droplets whose diameters may range from 1 to 100 $\mu$m.

2. A measuring device according to claim 1, wherein the polymer membrane is made of silicone rubber.

3. A measuring device according to claim 1, wherein the polymer membrane is made of a polymerized silicone prepolymer to which — in addition to the indicator droplets fluorescing in accordance with the pH-value — is bonded another indicator substance in solubilized form which is dispersed largely homogeneously and will fluorescence depending on the amount of $O_2$ present, and wherein the two indicators have different emission wavelengths.

4. A measuring device according to claim 3, wherein polycyclic, homocyclic or heterocyclic aromatic molecules, preferably polycyclic aromatic hydrocarbons with fluorescence decay times of $\tau_0$ greater than 5 ns are used as an $O_2$-dependent, fluorescent indicator.

5. A measuring device according to claim 3 or 4, wherein for the purpose of immobilization the $O_2$-dependent, fluorescent indicator is supplied in chemically modified form, preferably alkylated with longer hydrocarbon chains from $C_3$ to $C_{20}$, branched or unbranched, such that the indicator mobility in the silicone polymer is reduced.

6. A measuring device according to any of claims 1-5, wherein the polymer membrane is vulcanized or grafted onto a smooth surface of a transparent layer of carrier material.

7. A measuring device according to any of claims 1-6, wherein the polymer membrane material is provided with « place-holders » for the droplets containing the indicator substance, which have the same size as the droplets and which either keep the indicator solution on the surface or are impregnated by it.

8. A measuring device according to claim 7, wherein pellets or minute particles of hygroscopic material, such as silica gel or polyacrylamide, are used as « place-holders ».

9. A measuring device according to claim 7, wherein pellets of porous material, such as glass beads, are used as « place-holders ».

10. A measuring device according to any of claims 1-9, wherein the side of the polymer membrane facing the sample is provided with a protective membrane, preferably by grafting.

11. A measuring device according to claim 10, wherein the protective membrane facing away from the fluorometric arrangement is opaque.

12. A measuring device according to any of claims 1-11, wherein the indicator solution contains an additional buffer system, preferably bicarbonate or phosphate buffer.

## Revendications

1. Appareil de mesure pour la détermination de la teneur en $CO_2$ d'un échantillon, avec comme indicateur, un colorant fluorescent dépendant du pH, qui est incorporé en solution aqueuse, dans une membrane polymère, et qui peut être mis en contact, au moins partiellement avec l'échantillon et un dispositif pour mesurer la lumière fluorescente émise par l'indicateur après excitation, appareil caractérisé en ce que la solution aqueuse d'indicateur se trouve incorporée sous la forme de gouttelettes d'un diamètre de l'ordre de 0,1 à 100 $\mu$m, en une répartition homogène, dans une membrane polymère perméable aux gaz et imperméable aux protons.

2. Appareil de mesure selon la revendication 1, caractérisé en ce que la membrane polymère est constituée par du caoutchouc de silicone repoussant l'eau.

3. Appareil de mesure selon la revendication 1, caractérisé en ce que la membrane est constituée par un polymère silicone dans lequel se trouve, en plus des gouttelettes d'indicateur fluorescent dépendant du pH, au moins une substance-indicateur, fluorescente en fonction de $O_2$, répartie d'une façon approximativement homogène, liée sous forme solubilisée, et que les deux indicateurs présentent des longueurs d'ondes d'émission différentes.

4. Appareil de mesure selon la revendication 3, caractérisé en ce que des molécules aromatiques polycycliques, homo- ou hérérocycliques, de préférence des hydrocarbures aromatiques polycycliques, avec une durée de retour à zéro de la fluorescence $\tau_0$ supérieure à 5 ns, sont utilisés comme indicateur fluorescent en fonction de $O_2$.

5. Appareil de mesure selon la revendication 3 ou 4, caractérisé en ce que l'indicateur fluorescent en fonction de $O_2$, afin qu'il soit immobilisé, se trouve sous une forme chimiquement modifiée, de préférence alcoylée avec des chaînes longues d'hydrocarbures ramifiées ou non, de $C_3$ à $C_{20}$, ce qui limite la mobilité de l'indicateur dans le polymère silicone.

6. Appareil de mesure selon l'une des revendications 1 à 5, caractérisé en ce que la membrane polymère est appliquée avec vulcanisation ou polymérisée sur la surface lisse d'un matériau de support transparent, lamellaire.

7. Appareil de mesure selon l'une des revendications 1 à 6, caractérisé en ce que dans la matière de la membrane polymère, il est prévu des fixateurs d'emplacement pour les gouttelettes contenant l'indicateur, du même ordre de grandeur que celles-ci, qui, soit portent la solution d'indicateur en surface, soit sont imprégnés de part en part par celle-ci.

8. Appareil de mesure selon la revendication 7, caractérisé en ce que les fixateurs d'emplacement sont constitués par des globules ou de très petites particules d'un matériau hygroscopique, comme par exemple un gel de silice ou un polyacrylamide.

9. Appareil de mesure selon la revendication 7, caractérisé en ce que les fixateurs d'emplacement sont constitués de globules de matière poreuse, par exemple des petites billes de verre.

10. Appareil de mesure selon l'une des revendications 1 à 9, caractérisé en ce que, sur la face de la membrane polymère tournée vers l'échantillon, il est appliqué une membrane de protection déposée hermétiquement par polymérisation, de préférence.

11. Appareil de mesure selon la revendication 10, caractérisé en ce que la membrane de protection qui est à l'opposé du dispositif de mesure de la lumière fluorescente est opaque à la lumière.

12. Appareil de mesure selon l'une des revendications 1 à 11, caractérisé en ce que la solution d'indicateur contient un système tampon supplémentaire, de préférence un tampon bicarbonate ou phosphate.